(19) Europäisches Patentamt
European Patent Office
Office européen des brevets

(11) **EP 4 596 011 A1**

(12) **EUROPEAN PATENT APPLICATION**
published in accordance with Art. 153(4) EPC

(43) Date of publication:
06.08.2025 Bulletin 2025/32

(21) Application number: 24822687.0

(22) Date of filing: 11.06.2024

(51) International Patent Classification (IPC):
*A61M 16/01* (2006.01)    *A61M 16/00* (2006.01)

(52) Cooperative Patent Classification (CPC):
(C-Sets available)
**A61M 16/12; A61M 16/00; A61M 16/01;
A61M 16/024; A61M 16/104;** A61M 16/0069;
A61M 16/0891; A61M 16/18; A61M 16/203;
A61M 16/209; A61M 16/22; A61M 2016/0027;
A61M 2016/0039; A61M 2202/0208;
A61M 2202/0283;                        (Cont.)

(86) International application number:
**PCT/CN2024/098486**

(87) International publication number:
**WO 2024/255737 (19.12.2024 Gazette 2024/51)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **16.06.2023  CN 202310731222**

(71) Applicant: **Shenzhen Mindray Bio-Medical
Electronics Co., Ltd.
Shenzhen, Guangdong 518057 (CN)**

(72) Inventors:
• **CAI, Kun
  Shenzhen, Guangdong 518057 (CN)**
• **BAO, Wang
  Shenzhen, Guangdong 518057 (CN)**
• **CHEN, Yiwei
  Shenzhen, Guangdong 518057 (CN)**
• **YU, Wenhao
  Shenzhen, Guangdong 518057 (CN)**

(74) Representative: **KIPA AB
Drottninggatan 11
252 21 Helsingborg (SE)**

(54) **ANESTHESIA MACHINE**

(57) The present invention provides an anesthesia machine. A second fresh gas branch thereof is in communication with an air compressing apparatus having an adjustable supply air flow, and the air compressing apparatus provides air to the second fresh gas branch, thereby eliminating the need to use an air cylinder as a backup gas source, and reducing management risks and costs. Oxygen and/or nitrous oxide provided by a first fresh gas branch, air provided by the second fresh gas branch and an anesthetic are mixed to obtain a first mixed gas. An anesthetic delivery apparatus delivers the first mixed gas to a breathing circuit, and then a ventilation control apparatus controls the breathing circuit to deliver the first mixed gas to a patient, thereby providing anesthesia breathing support for the patient. A detection apparatus detects a characteristic quantity that characterizes the air flow features of the second fresh gas branch; a processor adjusts the air flow provided by the air compressing apparatus according to the characteristic quantity, so that the air compressing apparatus does not need to operate at a full load, thereby increasing the use time of the air compressing apparatus.

FIG. 2

(52) Cooperative Patent Classification (CPC): (Cont.)
A61M 2205/3334; A61M 2205/502

C-Sets
A61M 2202/0208, A61M 2202/0007;
A61M 2202/0283, A61M 2202/0007

**Description**

TECHNICAL FIELD

The disclosure relates to a technical field of medical device, and in particular, relates to an anesthesia machine.

BACKGROUND

Anesthesia machine is configured to provide anesthetic and respiratory support for a patient. Modern anesthesia machine generally consists of a gas source, a flow meter, a vaporizer, a patient loop, an anesthesia ventilator and an anesthesia gas scavenging system (AGSS).

The gas source provides $O_2$(oxygen), Air and $N_2O$ (laughing gas) for a gas inhaled by a patient.

The flow meter controls an amount of flow and an oxygen concentration for a gas inhaled by a patient.

The vaporizer controls a concentration of anesthetic in a gas inhaled by a patient.

The patient loop delivers a fresh gas containing $O_2$ and anesthetic to a patient for inhalation, while receives a gas exhaled by the patient, so as to transfer excess waste gas to the AGSS, and reuses most of the exhaled gas after removing $CO_2$.

The anesthesia ventilator provides a respiratory support to a patient, controls a volume or pressure of a gas inhaled by a patient, as well as a respiratory rhythm.

The AGSS acquires an excess gas exhaled by a patient and is connected with a treatment of waste gas system of hospital for treatment of waste gas.

A gas source type of modern anesthesia machine includes $O_2$, Air, and $N_2O$. It is easy to understand that $O_2$ is indispensable, otherwise the patient will suffer from respiratory hypoxia. However, simply inhaling oxygen is risky. Studies have shown that a long-term inhalation of pure oxygen can lead to oxygen poisoning. The role of air is to decrease an oxygen concentration in a fresh gas, which is especially important during a long operation. $N_2O$ is a mild anesthetic gas, and its frequency of use varies greatly in different countries. The gas source can be connected through a hospital central gas supply system or provided by a high-pressure gas cylinder. High-pressure gas cylinder is generally used as a backup gas source. When the hospital central gas supply system malfunctions, the gas stored in the high-pressure gas cylinder can be used as a gas source for the anesthesia machine.

An actual situation is that some hospitals have poor conditions and do not have a central gas supply system, or a central air supply system. As safe anesthesia is increasingly advocated, using air to decrease an oxygen concentration of a gas inhaled by the patient is receiving more and more attention. In the absence of hardware facilities of central air supply system, air can only be provided by relying on an air cylinder as a backup gas source. But we know that an air cylinder is expensive, and a transportation, storage and management of high-pressure gas brings risks and increases costs. Even for medical institutions with a central air supply system, there is still the problem of using high-pressure air cylinder in a situation where the central air supply system malfunctions.

Therefore, the existing anesthesia machine that uses an air cylinder as a backup gas source has problems, such as a management risk and a high cost, and needs to be improved and modified.

SUMMARY

This disclosure mainly provides an anesthesia machine which does not need to use an air cylinder as a backup gas source, thereby reducing a management risk and cost.

One embodiment provides an anesthesia machine, including:

a first fresh gas branch, which is configured to provide oxygen and/or nitrous oxide;
a second fresh gas branch, which is connected with an air compression apparatus and configured to provide air; wherein an air flow amount which is provided by the air compression apparatus is adjustable;
wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;
a respiratory loop, which is configured to receive the first mixed gas which is outputted by the anesthetic delivery apparatus, and deliver the first mixed gas to a patient;
a detection apparatus, which is configured to detect a characteristic quantity which characterizes an air flowing characteristic of the second fresh gas branch; and
a processor, which is configured to adjust the air flow amount which is provided by the air compression apparatus, according to the characteristic quantity which is outputted by the detection apparatus.

One embodiment provides an anesthesia machine, including:

a first fresh gas branch, which is configured to provide oxygen and/or nitrous oxide;
a second fresh gas branch, which is connected with an air compression apparatus and configured to provide air; wherein an air flow amount which is provided by the air compression apparatus is adjustable;

wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;

a respiratory loop, which is configured to receive the first mixed gas which is provided by the anesthetic delivery apparatus;

a ventilation control apparatus, which is configured to control the respiratory loop to deliver the first mixed gas to a patient, so as to provide an anesthesia respiratory support for the patient;

a human-machine interaction apparatus, which is configured to receive a target setting value for flow amount;

a processor, which is configured to adjust the air flow amount which is provided by the air compression apparatus, according to the target setting value for flow amount.

One embodiment provides an anesthesia machine, including:

a first fresh gas branch, which is configured to provide oxygen and/or nitrous oxide;

a second fresh gas branch, which is connected with an air compression apparatus and configured to provide air; wherein an air flow amount which is provided by the air compression apparatus is adjustable;

a second valve, which is configured to support a user to manually adjust an air flow amount which is provided by the second fresh gas branch;

wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;

a respiratory loop, which is configured to receive the first mixed gas which is outputted by the anesthetic delivery apparatus, and deliver the first mixed gas to a patient;

a detection apparatus, which is configured to detect a characteristic quantity of opening degree for the second valve; and

a processor, which is configured to adjust an output capacity of the air compression apparatus, according to a change of the characteristic quantity of opening degree.

One embodiment provides an anesthesia machine, including:

a first fresh gas branch, which is configured to provide oxygen and/or nitrous oxide;

a second fresh gas branch, which is connected with an air compression apparatus and configured to provide air;

wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;

a respiratory loop, which is configured to receive the first mixed gas which is outputted by the anesthetic delivery apparatus; and

a drive gas branch;

wherein one end of the drive gas branch is connected with the air compression apparatus, and the other end of the drive gas branch is connected with the respiratory loop; wherein the drive gas branch is configured to use air, which is provided by the air compression apparatus, as a drive gas, so as to periodically drive the respiratory loop to deliver the first mixed gas to a patient; or, the anesthesia machine further includes another air compression apparatus, wherein one end of the drive gas branch is connected with said another air compression apparatus, and the other end of the drive gas branch is connected with the respiratory loop; wherein the drive gas branch is configured to use air, which is provided by said another air compression apparatus, as a drive gas, so as to periodically drive the respiratory loop to deliver the first mixed gas to a patient.

According to the anesthesia machine of the above embodiment, its second fresh gas branch is connected with an air compression apparatus with an adjustable air flow amount, and the air compression apparatus provides air to the second fresh gas branch without requiring an air cylinder as a backup air source, which reduces a management risk and cost. Furthermore, the detection apparatus detects a characteristic quantity which characterizes an air flowing characteristic of the second fresh gas branch; the processor is further configured to adjust the air flow amount which is provided by the air compression apparatus according to the characteristic quantity, so that the air compression apparatus does not need to work at a full load, thereby increasing a service life of the air compression apparatus.

BRIEF DESCRIPTION OF THE DRAWINGS

FIG. 1 is a structural block diagram of an anesthesia machine according to an embodiment of this disclosure.

FIG. 2 is a structural block diagram of an anesthesia machine according to an embodiment of this disclosure.

FIG. 3 is a gas path diagram of an air compression apparatus and a portion of a second fresh gas branch in an anesthesia machine provided by this disclosure.

FIG. 4 is a working process of an anesthesia machine according to an embodiment of this disclosure.

FIG. 5 is a flow chart of an embodiment of step 3 in FIG. 4.

FIG. 6 is a structural block diagram of a detection apparatus according to an embodiment in an anesthesia machine provided by this disclosure.

FIG. 7 is a structural block diagram of an air compression apparatus, a ventilation control apparatus and a respiratory loop according to an embodiment in an anesthesia machine provided by this disclosure.

FIG. 8 is a structural block diagram of another air compression apparatus, a ventilation control apparatus and a respiratory loop according to an embodiment in an anesthesia machine provided by this disclosure.

FIG. 9 is a structural block diagram of an anesthesia machine according to an embodiment of this disclosure.

FIG. 10 is a structural block diagram of an anesthesia machine according to another embodiment of this disclosure.

DESCRIPTION OF THE PREFERRED EMBODIMENTS

This disclosure will be further described in detail below through specific embodiments in combination with the accompanying drawings. In different embodiments, similar elements adopt associated similar reference numbers. In the following embodiments, many details are described so as to make this disclosure better understood. However, one skilled in the art can easily recognize that some of the features can be omitted in different cases, or can be replaced by other elements, materials and methods. In some cases, some operations related to this disclosure are not shown or described in the description, so as to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the description may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The serial numbers assigned to the components in this article, such as "first", "second", etc., are only used to distinguish the objects described and do not have any order or technical meaning. In addition, the terms "connection", and "linkage" mentioned in this disclosure include direct and indirect connection (linkage), unless otherwise specified.

This disclosure integrates a fresh gas branch inside an anesthesia machine, which branch is connected with an air compression apparatus and air can be provided by the air compression apparatus. The anesthesia machine of this disclosure can realize fresh gas output and air output without requiring an external central gas supply system or a high-pressure gas cylinder. The anesthesia machine of this disclosure can also be used as a backup gas source to replace a high-pressure air cylinder, with basically no management risk and low cost. The following describes in detail through some specific embodiments.

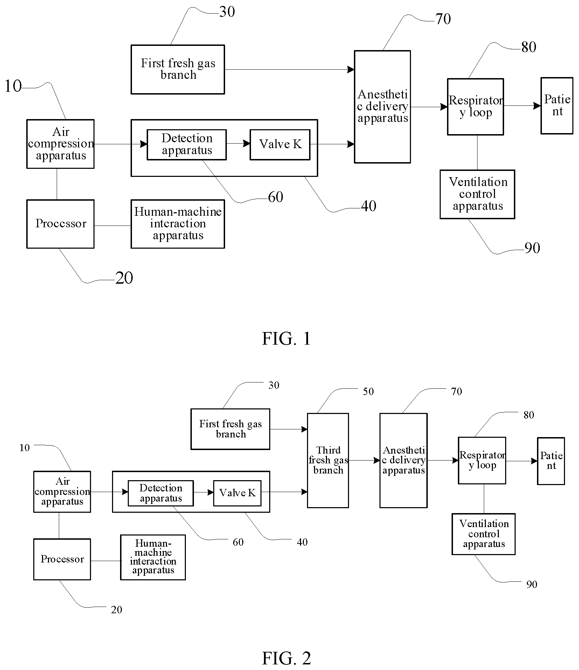

As shown in FIGS. 1 and 2, an anesthesia machine provided by this disclosure includes a processor 20, a first fresh gas branch 30, a second fresh gas branch 40, a respiratory loop 80, a ventilation control apparatus 90 and a detection apparatus 60.

The first fresh gas branch 30 is configured to provide oxygen and/or nitrous oxide. The first fresh gas branch 30 may include an oxygen interface and/or a nitrous oxide interface. The oxygen interface is connected with an external oxygen source or an internal oxygen source. An external oxygen source, such as an oxygen pipeline or oxygen cylinder in a central gas supply system, can be connected with the oxygen interface to provide oxygen to the anesthesia machine. The internal oxygen source may include an oxygenerator, etc., which provides oxygen to the anesthesia machine by docking with the oxygen interface. An external gas source of nitrous oxide, such as a nitrous oxide pipeline or a nitrous oxide cylinder in a central gas supply system, can be connected with a nitrous oxide interface to provide nitrous oxide to the anesthesia machine. The first fresh gas branch 30 may further include one or more valves, some of which are configured to adjust a flow amount of the oxygen, and some of which are used to adjust a flow amount of the nitrous oxide.

The second fresh gas branch 40 is connected with an air compression apparatus 10. For example, the second fresh gas branch 40 can be directly connected with an output end of the air compression apparatus 10, or can be indirectly connected with an output end of the air compression apparatus 10, as long as the second fresh gas branch 40 can be connected with the output end of the air compression apparatus 10. The second fresh gas branch 40 is configured to provide air, such as provide air to an anesthetic delivery apparatus 70 at a back end. The air compression apparatus 10 is configured to compress air, for example, to obtain and compress air from an external environment, and provide the compressed air to the second fresh gas branch 40. An output capacity of the air compression apparatus 10 is adjustable, for example, an air flow amount which is provided or outputted by the air compression apparatus 10 is adjustable. The air compression apparatus 10 can be arranged inside a housing of the anesthesia machine or can be placed outside a housing of the anesthesia machine. This embodiment is described using the former as an example, that is, in this embodiment, the anesthesia machine includes or has a built-in air compression apparatus 10.

The anesthesia machine may also include an air interface. The air interface is configured to receive air provided from outside, such as to connect with an external air source. The external air source, such as an air pipe in a central air supply system, can be ducked with the air interface, and configured to provide air to the anesthesia machine. Either the air interface or the air compression apparatus 10 is connected with the second fresh gas branch 40. In one usage scenario, the anesthesia machine is used in a hospital and the air pipe in the hospital can be connected with the air interface. The anesthesia machine obtains fresh air from a central air supply system in the hospital, and the air compression apparatus 10 serves as a backup air source. When the central air supply system in the hospital malfunctions or the anesthesia machine is used outside the hospital, the air compression apparatus 10 is started to provide air.

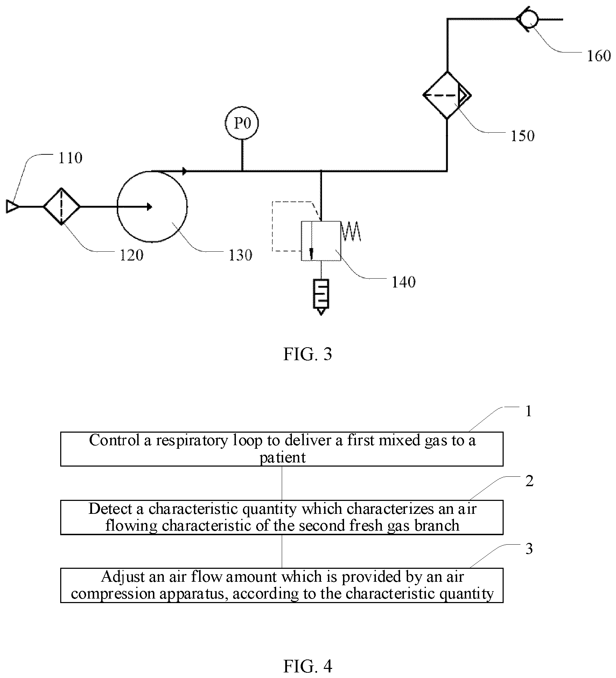

The air compression apparatus 10 can be of various types according to different air compression methods. For example, air is compressed by a rotation of blades, or by a reciprocation movement of a piston. Therefore, an output capacity of the air compression apparatus 10 being adjustable, can be for example a speed of the rotation, a stroke or a frequency of the reciprocation movement can be adjustable. From a perspective of work done, an output power is adjustable, and from a perspective of effect, an air flow amount provided (outputted) is adjustable. As shown in FIG. 3, in one embodiment, the air compression apparatus 10 includes an air pump 130 with an adjustable rotation speed. Of course, in other embodiments, the air compression apparatus 10 may also include a turbine or an air compressor, etc. The anesthesia machine may have an air inlet 110, a first filter 120, and a second filter 150. Wherein the air inlet 110, the first filter 120, the air pump 130 and the second filter 150 are sequentially connected with each another.

The air inlet 110 is connected with atmosphere. When the air pump 130 is working, external air enters into the air compression apparatus 10 from the air inlet 110 after being filtered by the first filter 120. The air pump 130 is configured to compress the air entering through the air inlet 110 and output the compressed air to the second fresh gas branch 40. The first filter 120 is arranged between the air inlet 110 and the air pump 130, and is configured to filter air that is about to enter into the air pump 130. The second filter 150 is configured to filter the air which is outputted by the air pump 130. For example, its filtration accuracy can reach 0.3 um to satisfy a requirement of patient for inhalation. A one-way valve 160 may also be provided inside the second fresh gas branch 40. The one-way valve 160 is arranged after the second filter 150 and is used for outputting the air filtered by the second filter 150 in a one-way manner.

A normally closed pressure-relief branch may also be provided at the second fresh gas branch 40, such as a branch where 140 is located in FIG. 3. The branch may be provided, for example, between the air pump 130 and the second filter 150. The normally closed pressure-relief branch includes an overflow valve 140, which is arranged at a back end of the air pump 130. The overflow valve 140 is configured to open the normally closed pressure-relief branch when an air pressure of the second fresh gas branch 40 is higher than a work pressure, so as to enable the air inside the second fresh gas branch 40 to flow out from the normally closed pressure-relief branch.

The built-in air pump 130 inhales air from the air inlet 110 and filters the air through the first filter 120, so as to protect the air pump 130 and back-end components. After the air is pressurized by the air pump 130, a pressure sensor P0 and the overflow valve 140 are connected at the back end of the air pump 130. The pressure sensor P0 is configured to monitor an air pressure at the back end of the air pump 130. The overflow valve 140 can prevent the air pressure at the back end of the air pump 130 from exceeding a preset work pressure of the overflow valve 140. The overflow valve 140 can be electronic or mechanical. Mechanical overflow valve can better resist an electronic control malfunction of the anesthesia machine. When the air pressure at the back end of the air pump 130 exceeds the preset work pressure, the overflow valve 140 opens, and part of the air inside the second fresh gas branch 40 is discharged into the atmosphere through the overflow valve 140 to decrease the air pressure. When an electronic overflow valve is used, the preset work pressure can be a system preset value for pressure determination. When a mechanical overflow valve is used, the preset work pressure is a valve sealing pressure for a valve plate of the mechanical overflow valve. When the air pressure is greater than the valve sealing pressure, the valve plate will be pushed to open, so as to allow the air to flow out of the overflow valve. The second filter 150 is configured to further filter the air at the back end of the air pump 130. The one-way valve 160 prevents the air from flowing back, and prevents the air inside the second fresh gas branch 40 from flowing out of the air pump 130 when the air pump 130 is not started.

The detection apparatus 60 is configured to detect a characteristic quantity that characterizes an air flowing characteristic of the second fresh gas branch 40. That is, the characteristic quantity is capable of reflecting a flowing characteristic of air inside the second fresh gas branch 40. The flow characteristic may be a flow amount, a flow velocity or a pressure, etc., which is associated with a size of an air flow amount of the second fresh gas branch 40. The characteristic quantity may be the air flow amount itself, or a factor which affects the air flow amount, etc., as long as it can reflect the air flowing characteristic of the second fresh gas branch 40, and this disclosure is not limited thereto.

The first fresh gas branch 30 and the second fresh gas branch 40 are connected with the anesthetic delivery apparatus 70. For example, FIG. 1 and FIG. 2 show two connection modes. The anesthetic delivery apparatus 70 can be used as an external device applicable to the anesthesia machine, or can be a part of the anesthesia machine. The anesthetic delivery apparatus 70 contains anesthetic, and is configured to mix the gas provided by the first fresh gas branch 30, the air provided by the second fresh gas branch 40, and the anesthetic, so as to obtain a first mixed gas, and deliver the first mixed gas to the respiratory loop 80. Wherein, the gas (such as oxygen and/or nitrous oxide) provided by the first fresh gas branch 30 and the air provided by the second fresh gas branch 40 can be mixed in the anesthetic delivery apparatus 70, or can be mixed firstly and then mixed with the anesthetic in the anesthetic delivery apparatus 70. This embodiment is described using the latter as an example. As shown in FIG. 2, the anesthesia machine further includes a third fresh gas branch 50. The third fresh gas branch 50 is configured to mix the gas provided by the first fresh gas branch 30 and the air provided by the second fresh gas branch 40, so as to obtain a second mixed gas (such as an oxygen-air mixed gas, a nitrous oxide-air mixed gas, or an oxygen-nitrous oxide-air mixed gas, etc.). The anesthetic delivery apparatus 70 is connected with the third fresh gas branch 50, and is configured to mix the second mixed gas with the anesthetic to obtain the first mixed gas, and further control an anesthetic concentration of the first mixed gas. The anesthetic delivery apparatus 70 may include a vaporizer.

The respiratory loop 80 is a gas path which is connected the anesthetic delivery apparatus 70 with a patient, and is configured to deliver the first mixed gas to the patient. The respiratory loop 80 can recycle a gas exhaled by the patient to save anesthetic and reduce environmental pollution. The respiratory loop 80 may include various connection tubes and output ports. The output ports may be various types of accessories. The accessories may be an endotracheal tube, an endotracheal tube with an air bag at its end, and the likes. A gas purification apparatus may be provided inside the respiratory loop 80, and the gas purification apparatus is configured to remove at least part of carbon dioxide exhaled by the patient into the respiratory loop. For example, $CO_2$ absorbent (soda lime) can be provided inside the gas purification apparatus. The purpose of removing $CO_2$ is achieved by reacting the $CO_2$ absorbent with $CO_2$. At the same time, the reaction generates water and heat, which is beneficial to maintaining a temperature and humidity of the gas inhaled by the patient.

The ventilation control apparatus 90 is configured to control the respiratory loop 80 to deliver the first mixed gas to the patient through its output port, so as to enable the anesthesia machine to provide an anesthetic respiratory support for the patient. For example, the respiratory loop 80 is controlled to periodically deliver the first mixed gas to the patient, so as to enable the anesthesia machine to provide a periodic anesthetic respiratory support for the patient. The ventilation control apparatus 90 can perform an anesthesia ventilation control automatically or manually (such as a balloon). For example, the ventilation control apparatus 90 may include multiple valves and a board for driving the multiple valves. The board controls the multiple valves to periodically deliver the first mixed gas to the patient, so as to provide a periodic anesthesia respiratory support for the patient.

The processor 20 is configured to adjust the output capacity of the air compression apparatus 10 according to the characteristic quantity outputted by the detection apparatus 60, such as adjusting the air flow amount (outputted) provided by the air compression apparatus 10. The air flow amount outputted by the air compression apparatus 10 is adjusted based on the air flowing characteristic of the second fresh gas branch. This can assist in a flow amount adjustment, and also allow the air compression apparatus 10 to operate at a load which is less than a full load, thereby increasing a service life of the air compression apparatus, indirectly saving cost and reducing a noise caused by the use of the air compression apparatus 10.

As shown in FIG. 4, a working process of the anesthesia machine provided by this disclosure may include following steps.

Step 1: After an anesthesia machine is started, an air compression apparatus 10 is turned on, and each fresh gas branch is also connected. Each fresh gas branch provides a corresponding fresh gas, and various fresh gases are added with anesthetic and a concentration of the anesthetic is adjusted through a vaporizer to form a first mixed gas; the first mixed gas enters into a respiratory loop 80, and a ventilation control apparatus 90 performs a ventilation control to deliver the first mixed gas to a patient, and a waste gas exhaled by the patient is purified by a gas purification apparatus and then discharged or recycled; during the above process, the anesthesia machine will also monitor a machine state and a patient parameter to ensure patient safety and issue an abnormal alarm.

Step 2: During an operation of the anesthesia machine, a detection apparatus 60 detects a characteristic quantity that characterizes an air flowing characteristic of a second fresh gas branch 40.

Step 3: A processor 20 adjusts an air flow amount which is provided by the air compression apparatus 10, according to the characteristic quantity which is detected by the detection apparatus 60.

A valve K may be provided at the second fresh gas branch 40 or the third fresh gas branch 50 to adjust the air flow amount. There are two main types of value adjustment: one is a manual needle-valve adjustment, and the other is an electromagnetic valve control adjustment. Both adjustment methods adjust a diameter of the fresh gas branch to achieve the purpose of adjusting the flow amount. When ways for adjusting the flow amount are different, ways for detecting the characteristic quantity by the detection apparatus 60 may be different. Several embodiments are given below to illustrate.

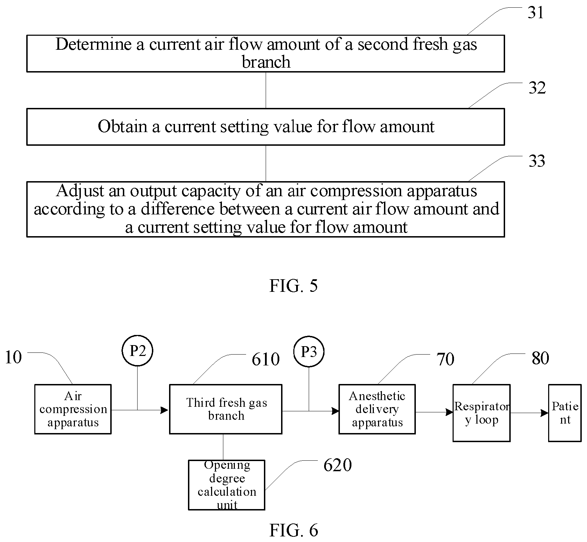

In a first embodiment, the anesthesia machine further includes a first valve which arranged at the second fresh gas branch 40, such as the valve K in FIGS. 2 and 3. The first valve is configured to adjust an air flow amount inside the second fresh gas branch 40. As shown in FIG. 5, step 3 may include the following steps.

Step 31: The processor 20 determines a current air flow amount of the second fresh gas branch 40. The processor 20 may specifically obtain the current air flow amount directly from the detection apparatus 60, or may indirectly calculate the current air flow amount based on the characteristic quantity. The two methods are described below.

In the first manner, the characteristic quantity is the current air flow amount of the second fresh gas branch 40. For example, the detection apparatus 60 includes a first flow amount sensor. The first flow amount sensor may be arranged inside the second fresh gas branch 40. The first flow amount sensor is configured to detect the current air flow amount of the second fresh gas branch 40, and the current air flow amount detected by the first flow amount sensor is used as the characteristic quantity. There are many monitoring principles for flow amount sensor, including a pressure difference principle, a hot wire principle and an ultrasound principle. The first flow amount sensor may be arranged inside the second fresh gas branch 40. If the first valve adopts an electromagnetic valve, because the processor 20 needs to control the electromagnetic valve to set an air flow amount, the electromagnetic valve will be associated with the first flow amount sensor. Usually, the first flow amount sensor and the electromagnetic valve are arranged inside the anesthesia machine in a form of a full electronic flow meter. The processor 20 may receive an current air flow amount outputted by the first flow amount sensor, so as to control an output capacity of the air compression apparatus 10 (e.g., to control a rotation speed of the air pump 130).

In the second manner, the characteristic quantity does not include the current air flow amount of the second fresh gas branch. The processor 20 obtains the current air flow amount of the second fresh gas branch 40 according to the characteristic quantity outputted by the detection apparatus 60. For example, the anesthesia machine further includes an oxygen concentration sensor for detecting an oxygen concentration of the second mixed gas. The detection apparatus 60 includes a second flow amount sensor. The second flow amount sensor is configured to detect a flow amount of a gas which is provided by the first fresh gas branch 30, or to detect a mixed gas flow amount of the second mixed gas which gas is provided by the third fresh gas branch 50.

If the gas provided by the first fresh gas branch does not contain nitrous oxide, such as the first fresh gas branch only provides oxygen, the processor 20 can calculate the current air flow amount of the second fresh gas branch 40, according to the oxygen concentration of the second mixed gas and a flow amount of oxygen which oxygen is provided by the first fresh gas branch, or according to an oxygen concentration of the second mixed gas and a flow amount of the second mixed gas which gas is provided by the third fresh gas branch, so as to adjust an output capacity of the air compression apparatus 10 (such as adjusting a rotation speed of the air pump 130).

The first method is simple and convenient, and this embodiment is described by taking the first method as an example. After the processor 20 obtains a current air flow amount, it can also store current air flow amounts at different time points, and can also display the current air flow amounts through a human-machine interaction apparatus, so as to enable a doctor to view and monitor an air flow amount.

Step 32: The processor 20 obtains a current setting value for flow amount of the second fresh gas branch. In this embodiment, the anesthesia machine further includes a human-machine interaction apparatus. The human-machine interaction apparatus is used for human-machine interaction, for example, for displaying visual information and receiving a user input, and may include a display, a touch screen, button(s), a keyboard, a mouse, etc. The processor 20 may receive a setting value for flow amount of the second fresh gas branch, which value is inputted by a user through the human-machine interaction apparatus.

Step 33: The processor 20 adjusts an output capacity of the air compression apparatus 10 according to a difference between a current air flow amount of the second fresh gas branch and a current setting value for flow amount of the second fresh gas branch. Specifically, when the current air flow amount is lower than the current setting value for flow amount, the processor 20 increases the output capacity of the air compression apparatus 10, so as to increase an air flow amount, that is, to increase an air flow amount provided by the air compression apparatus. Taking a size of the output capacity of the air compression apparatus as a rotation speed of the air pump 130 as an example, the rotation speed of the air pump 130 is specifically increased. When the current air flow amount is higher than the current setting value for flow amount, the processor 20 decreases the output capacity of the air compression apparatus 10 to decrease the air flow amount, that is, to decrease the air flow amount provided by the air compression apparatus, specifically to decrease the rotation speed of the air pump 130.

The air pump 130 generates noise when working and has a problem with its service life. For the air pump 130 with an adjustable rotation speed, the higher the rotation speed, the louder the noise and the shorter the service life. In this embodiment, the rotation speed of the air pump 130 is adjusted according to an actual fresh air flow amount. It is easy to understand that a low speed can satisfy a requirement of a small flow amount, and a large air flow amount requires a higher air pump speed. In this case, the air pump 130 does not need to operate at the highest speed (full load) under any circumstances. Today, as people pay more and more attention to low-flow anesthesia, a low-flow fresh gas is used more and more, so in most cases, the air pump 130 only needs to work at a low speed. Obviously, this method can effectively increase the service life of the air pump 130 and reduce a working noise.

The processor 20 is capable of adjusting the output capacity of the air compression apparatus 10 alone to make the difference between the current air flow amount and the current setting value for flow amount approach 0 (i.e., adjust the current air flow amount to be the current setting value for flow amount). Optionally, the processor 20 is capable of adjusting both the first valve and the output capacity of the air compression apparatus 10, so as to make the difference between the current air flow amount and the current setting value for flow amount approach 0. This embodiment is described using the latter as an example.

In this embodiment, the first valve is an electromagnetic valve, that is, the processor 20 can control an opening degree of the first valve without requiring a doctor to manually twist the valve. Therefore, in step 32, after receiving a setting value for flow amount inputted by the user through the human-machine interaction apparatus, the processor 20 controls the opening degree of the first valve to adjust the air flow amount of the second fresh gas branch 40 to the setting value for flow amount.

After the doctor inputs a new setting value for flow amount, a difference between a current air flow amount and a current (new) setting value for flow amount will be relatively large, so the processor 20 can also adjust the opening degree of the first valve according to the difference between the current air flow amount and said current setting value for flow amount. The processor 20 can first adjust the opening degree of the first valve, and then adjust the output capacity of the air compression apparatus 10. Specifically, when a current air flow amount is lower than a current setting value for flow amount (the difference at this time is relatively large), as a primary measure, the processor 20 increases the opening degree of the first valve to increase an air flow amount, according to the difference between the current air flow amount and the current setting value for flow amount, and then re-acquires a current air flow amount. As a secondary measure, the processor 20 increases a rotation speed of the air pump 130 to increase an air flow amount, according to a difference between the re-acquired current air flow amount and the current setting value for flow amount, because the valve opening has been adjusted, said difference at this time is relatively small. When a current air flow amount is higher than the current setting value for flow amount, as a primary measure, the processor 20 decreases the opening degree of the first valve to decrease an air flow amount, according to a difference between said current air flow amount and the current setting value for flow amount, and then re-acquires a current air flow amount. As a secondary measure, the processor 20 decreases a rotation speed of the air pump 130 to decrease an air flow amount, according to a difference between the re-acquired current air flow amount and the current setting value for flow amount. This not only can quickly adjust a current air flow amount to the current setting value for flow amount, but is also very friendly to the service life of the air pump. Of course, the processor can also adjust the opening degree of the first valve and the output capacity of the air compression apparatus at the same time. The processor can also first adjust the output capacity of the air compression apparatus and then adjust the opening degree of the first valve. Specifically, when a current air flow amount is lower than a current setting value for flow amount (a difference at this time is relatively large), as a primary measure, the processor 20 increases the rotation speed of the air pump 130 to increase an air flow amount according to the difference between said current air flow amount and the current setting value for flow amount, and then re-acquires a current air flow amount. As a secondary measure, the processor 20 increases the opening degree of the first valve to increase an air flow amount, according to a difference between the re-acquired current air flow amount and the current setting value for flow amount, because the rotation speed has been adjusted, said difference at this time is relatively small. When a current air flow amount is higher than a current setting value for flow amount, the processor 20 decreases the rotation speed of the air pump 130 to decrease an air flow amount, according to a difference between said current air flow amount and said current setting value for flow amount, and then re-acquires a current air flow amount, and decreases the opening degree of the first valve to decrease an air flow amount, according to a difference between the re-acquired current air flow amount and the current setting value for flow amount.

Embodiment 2: In this embodiment, the anesthesia machine further includes: a second valve which is arranged inside the second fresh gas branch 40, such as the valve K shown in FIGS. 2 and 3, and the second valve is configured to support the user to manually adjust an air flow amount of the second fresh gas branch 40. Compared with setting the air flow amount by the electromagnetic valve in the first embodiment, the air flow in this embodiment needs to be manually adjusted by a doctor.

A mechanical flow meter or an electronic flow meter (such as a flow amount sensor) may also be provided inside the second fresh gas branch 40. Wherein, the mechanical flow meter has an instrument that displays an amount size of flow, or has a glass tube that displays an amount size of flow, wherein the amount size of flow is reflected by a position of a float inside the glass tube, and there are corresponding scale values on the glass tube to display a specific value of flow amount. The doctor can adjust the second valve according to a real-time air flow amount detected by the mechanical flow meter and a patient condition. There are many monitoring principles for a flow amount sensor, including a pressure difference principle, a hot wire principle and an ultrasound principle. An air flow amount can be monitored by a flow amount sensor, and data can be transmitted to the processor 20, which stores the data and displays the air flow amount through a display interface of the human-machine interaction apparatus.

Since the air flow amount is completely controlled manually by the doctor, the processor 20 cannot know an air flow amount that the doctor wants (corresponding to the setting value for flow amount of the previous embodiment). Therefore, in this embodiment, the characteristic quantity uses a characteristic quantity of opening degree for the second valve. The processor 20 adjusts an air flow amount which is provided by the air compression apparatus, according to a change of the characteristic quantity, which change is caused by a user via manually adjusting the second valve; that is, the processor obtains a trend of change for the characteristic quantity of opening degree according to the characteristic quantity of opening degree for the second valve, and then adjusts the air flow amount which is provided by the air compression apparatus 10 according to the trend of change for the characteristic quantity of opening degree. Specifically, when the change of the characteristic quantity of opening degree indicates an increase of the opening degree, that is, when the trend of change for the characteristic quantity of opening degree is an increase of the opening degree, it means that the doctor wants to increase the air flow amount, so that the processor 20 increases an output capacity of the air compression apparatus 10 to increase the air flow amount. When the change of the characteristic quantity of opening degree indicates a decrease of the opening degree, that is, when the trend of change for the characteristic quantity of opening degree is a decrease of the opening degree, it means that the doctor wants to decrease the air flow amount, so that the processor 20 decreases the output capacity of the air compression apparatus 10 to decrease the air flow amount. In this way, it can not only assist in adjusting the air flow amount, but also reduce a workload of the air compression apparatus 10 when a low-flow air supply becomes a trend, thereby extending a service life of the air compression apparatus 10.

From the above content, it can be seen that in this embodiment, it is particularly important that the detection apparatus detects the characteristic quantity of opening degree for the second valve, and then the processor is further configured to adjust the output capacity of the air compression apparatus accordingly. There are many specific implementation methods, and several examples are given below for illustration.

In a first embodiment, the detection apparatus 60 includes a first pressure sensor. The first pressure sensor can be arranged between the air compression apparatus and the second valve, can be arranged adjacent to a back end of the air compression apparatus, and can also be arranged adjacent to the second valve, that is, the first pressure sensor is configured to detect an air pressure of the second fresh gas branch 40 at a front end of the second valve. That is, the characteristic quantity of opening degree for the second valve is the air pressure detected by the first pressure sensor. This method does not require to refer to the air flow amount, so the anesthesia machine can use a mechanical flow meter to monitor the air flow amount.

The processor 20 obtains a trend of change for the air pressure according to air pressures at different time points. When the trend of change for the air pressure is an increase of the air pressure, it means that the doctor has turned down the second valve, so the processor 20 decreases the output capacity of the air compression apparatus 10; when the trend of change for the air pressure is a decrease of the air pressure, it means that the doctor has turned up the second valve, so the processor 20 increases the output capacity of the air compression apparatus 10.

In this embodiment, adjusting the output capacity of the air compression apparatus 10 may be adjusting the rotation speed of the air compression apparatus 10. For example, different air pressures can be matched with different rotation speeds in advance. The air pressure is inversely proportional to the rotation speed. The processor 20 increases and decreases the rotation speed of the air compression apparatus 10, such that the rotation speed of the air compression apparatus 10 can be adjusted to a rotation speed corresponding to a current air pressure. Since the higher the air pressure, the lower the rotation speed, and the lower the air pressure, the higher the rotation speed, the above-mentioned adjustment can be achieved. Of course, an interval control can also be used, for example, different pressure intervals are matched with different rotation speeds in advance, and an average air pressure in the pressure interval is inversely proportional to a corresponding rotation speed, that is, the higher the pressure interval, the lower the rotation speed, and the lower the pressure interval, the higher the rotation speed. Increasing or decreasing the rotation speed of the air compression apparatus 10 by the processor 20 can be that the processor 20 obtains a pressure range to which a current air pressure belongs according to the current air pressure, and then adjusts a rotation speed of the air compression apparatus 10 to a rotation speed which corresponds to the pressure range, which can also achieve the above-mentioned adjustment.

Some anesthesia machines do not have a flow amount sensor. To solve the problem of identifying a flow amount in this case, this disclosure creatively identifies the flow amount by monitoring a pressure at a back end of the air pump. When the air flow amount is adjusted from small to large, the pressure at the back end of the air pump will decrease; when the air flow amount is adjusted from large to small, the pressure at the back end of the air pump will increase. This method senses an adjustment of a user for the air flow amount through a change of the pressure at the back end of the air pump. When the required gas flow amount is detected to be increased, the rotation speed of the air pump can be increased to satisfy the requirement; when the required gas flow amount is detected to be decreased, the rotation speed of the air pump can be decreased. Of course, this method is also applicable to an anesthesia machine that adjusts the air flow amount through an electromagnetic valve control (Example 1) and a semi-electronic anesthesia machine (mechanically adjusts the air flow amount and uses a flow amount sensor to monitor and display the air flow amount).

In the second embodiment, as shown in FIG. 6, the detection apparatus 60 includes a second pressure sensor P2, a third flow amount sensor 610, and a third pressure sensor P3. The second pressure sensor P2, the third flow amount sensor 610 and the third pressure sensor P3 are sequentially arranged at the second fresh gas branch 40.

The third flow amount sensor 610 is configured to detect an air flow amount of the second fresh gas branch 40. The processor 20 is also configured to store the air flow amount and display the air flow amount through the display interface of the human-machine interaction apparatus, so that the doctor can monitor the air flow amount. The second valve may be arranged before the third flow amount sensor 610 or after the third flow amount sensor 610.

The second pressure sensor P2 is configured to detect an air pressure of the second fresh gas branch at a front end of the second valve, that is, to detect an air pressure between the air compression apparatus 10 and the second valve, that is, to detect an air pressure at the front end of the second valve.

The third pressure sensor P3 is configured to detect an air pressure at the back end of the second valve. That is, pressure sensors P2 and P3 are located before and after the second valve respectively, that is, the second valve is located between the second pressure sensor P2 and the third pressure sensor P3, so that after the opening degree for the second valve is adjusted, the pressure difference between the second pressure sensor P2 and the third pressure sensor P3 can be reflected.

The air pressure detected by the second pressure sensor, the air flow amount detected by the third flow amount sensor, and the air pressure detected by the third pressure sensor can be used to calculate the characteristic quantity of opening degree for the second valve. Specifically, the characteristic quantity of opening degree for the second valve can be calculated by an opening degree calculation unit 620 of the detection apparatus 60, or can be calculated by the processor 20.

The opening degree calculation unit 620 or the processor 20 is configured to receive the air flow amount detected by the third flow amount sensor 610, the air pressure detected by the second pressure sensor P2, and the air pressure detected by the third pressure sensor P3; and calculate the characteristic quantity of opening degree for the second valve based on the air pressure detected by the second pressure sensor P2, the air flow amount detected by the third flow amount sensor 610, and the air pressure detected by the third pressure sensor P3. Specifically, the opening degree calculation unit 620 or the processor 20 subtracts the air pressure detected by the third pressure sensor P3 from the air pressure detected by the second pressure sensor P2, so as to obtain a pressure difference, and divides the pressure difference by the air flow amount F detected by the second flow amount sensor 610, so as to obtain the characteristic quantity of opening degree for the second valve. In other words, the opening degree calculation unit 620 or the processor 20 can calculate the characteristic quantity of opening degree R according to the following formula R:

$$R = \frac{(P_2 - P_3)}{F}.$$

Wherein, R represents the characteristic quantity of opening degree, P2 represents the air pressure detected by the second pressure sensor, P3 represents the air pressure detected by the third pressure sensor, and F represents the air flow amount detected by the third flow amount sensor 610.

After the second valve adjusts the air flow amount, the method uses an actual air flow amount and a resulted pressure change to calculate the opening degree for the second valve, and the result is accurate and reliable.

In the third embodiment, the detection apparatus 60 includes a third flow amount sensor. The third flow amount sensor may be arranged inside the second fresh gas branch 40. The third flow amount sensor is configured to detect an air flow amount of the second fresh gas branch 40. The characteristic quantity of opening degree for the second valve is the air flow amount, or the air flow amount can be directly used as the characteristic quantity. When the air flow amount detected by the third flow amount sensor increases, the processor 20 increases the output capacity of the air compression apparatus 10; when the air flow amount detected by the third flow amount sensor decreases, the processor 20 decreases the output capacity of the air compression apparatus 10. The adjustment for the opening degree for the second valve and the adjustment for the rotation speed of the air pupm will affect the real-time air flow amount, accordingly, adjusting the rotation speed of the air pump simply relying on the real-time air flow is not as accurate as the previous method.

In the fourth embodiment, the detection apparatus 60 includes a displacement sensor or a gap sensor. The displacement sensor or the gap sensor is configured to detect the opening degree for the second valve to obtain the characteristic quantity of opening degree.

Specifically, the displacement sensor obtains the characteristic quantity of opening degree by detecting a relative position change between a needle valve and a valve seat of the second valve. The gap sensor may be, for example, an optical grating, which is configured to detect a size of a gap between the needle valve and the valve seat of the second valve to obtain the characteristic quantity of opening degree.

When the doctor uses the second valve of the needle valve structure to adjust the air flow amount, the relative position between the needle valve and the valve seat changes. A displacement sensor or a gap sensor is configured to monitor the relative position between the needle valve and the valve seat. When the relative position becomes larger, it means that the valve opening becomes larger, and the processor 20 increases the output capacity of the air compression apparatus 10; when the relative position becomes smaller, it means that the valve opening becomes smaller, and the processor 20 decreases the output capacity of the air compression apparatus 10. This method is also suitable for the anesthesia machine that uses a mechanical flow meter to detect the air flow amount.

Similarly, in this embodiment, the processor 20 may adjust the output capacity of the air compression apparatus 10 by adjusting the rotation speed of the air compression apparatus 10, such as adjusting the rotation speed of the air pump. In this embodiment, various methods of adjusting the rotation speed of the air pump can not only satisfy the demand for fresh gas, but also reduce the noise level of the air pump, while increasing the service life of the air pump.

In the above-mentioned embodiments, a pressure of a gas which is supplied by the anesthesia machine to the patient is stable, and what is adjusted or changed is a flow amount of supplied gas. In order to maintain the stability of the pressure of the supplied gas, no matter which embodiment is adopted and which method is configured to detect the characteristic quantity of the flow amount. The anesthesia machine can include a first pressure sensor (such as P0 in FIG. 3). The first pressure sensor is configured to detect an air pressure of the second fresh gas branch 40, that is, to detect the air pressure at the back end of the air compression apparatus 10.

The air pressure inside the second fresh gas branch is usually constant, for example, the air pressure needs to be maintained at a preset target pressure, or the air pressure is usually stable in a range, for example, the air pressure needs to be maintained in a preset target pressure range.

A target pressure and a target pressure range can be preset according to a requirement of user, or can be arranged inside system, which can ensure that when the air pressure is the target pressure or within the target pressure range, even if the opening degree of the first valve or the second valve reaches the maximum, the corresponding air flow amount still can exceed a preset minimum flow amount, and no condensed water will appear in the second fresh gas branch.

When the air pressure detected by the first pressure sensor is lower than the preset target pressure, the processor 20 can increase the output capacity of the air compression apparatus 10 to increase the air pressure, so as to maintain the air pressure at the target pressure; when the air pressure detected by the first pressure sensor is higher than the preset target pressure, the processor 20 can decrease the output capacity of the air compression apparatus 10 to decrease the air pressure, so as to maintain the air pressure the target pressure. When the air pressure detected by the first pressure sensor is lower than a lower limit of the target pressure range, the processor 20 increases the output capacity of the air compression apparatus 10 to increase the air pressure, so as to maintain the air pressure within the target pressure range; when the air pressure detected by the first pressure sensor is higher than an upper limit of the target pressure range, the processor 20 decreases the output capacity of the air compression apparatus 10 to decrease the air pressure, so as to maintain the air pressure within the target pressure range.

When the first pressure sensor at the back end of the air compression apparatus is configured to adjust the air flow amount which is provided by the air compression apparatus, the characteristic quantity of opening degree is the air pressure detected by the first pressure sensor. At this time, when a change of the characteristic quantity of opening degree (air pressure) indicates an increase of the opening degree, the processor increases the air flow amount provided by the air compression apparatus; when the change of the characteristic quantity of opening degree (air pressure) indicates a decrease of the opening degree, the processor decreases the air flow amount provided by the air compression apparatus. In addition to controlling according to a target pressure or a target pressure range, this disclosure can also directly adjust the air compression apparatus based on the change of the air pressure. When the air pressure decreases, increase the air flow amount provided by the air compression apparatus; when the air pressure increases, decrease the air flow amount provided by the air compression apparatus. For example, if the air pressure at a current moment becomes smaller than the air pressure at a previous moment or a previous time interval, the air pressure is considered to have been decreased and the air flow amount provided by the air compression apparatus needs to be increased. When the air pressure at a current moment becomes larger than the air pressure at a previous moment or a previous time interval, the air pressure is considered to have increased, and the air flow amount provided by the air compression apparatus needs to be decreased.

When the processor 20 adjusts the rotation speed of the air compression apparatus 10, the processor 20 can obtain the air pressure of the second fresh gas branch 40. When the air pressure is higher than a preset target pressure (such as a control target of the air pump), the rotation speed of the air compression apparatus 10 needs to be decreased, so as to decrease the output capacity, and the pressure at the back end of the air compression apparatus 10 needs to be passively released through a flow amount output. The inventors have discovered that due to an inevitable leakage of a certain size in various components of the air compression apparatus 10 and the anesthesia machine loop, the pressure at the back end of the air compression apparatus 10 slowly decreases when the adjustment valve of fresh gas (the first valve and the second valve mentioned above) is completely closed or opened to a small extent. At this time, a pressure release process at the back end of the air compression apparatus 10 is relatively long, and the air compression apparatus 10 may decrease its rotation speed to zero and shut down. When the pressure is released to below the target pressure, it is necessary to increase the rotation speed of the air compression apparatus 10 to increase the pressure at the back end. After the air compression apparatus 10 is shut down, when the pressure at the back end is lower than the target pressure, there is also a certain pressure at the back end of the air compression apparatus 10. If the air compression apparatus 10 increases its rotation speed from 0, since an output torque corresponding to a low rotation speed is also small, the air compression apparatus 10 needs a higher rotation speed to restart. At this time, since an opening degree of the back end is small and the pressure at the back end is easy to exceed the target pressure, the air compression apparatus 10 will start and stop repeatedly. This phenomenon of repeated starting and stopping is easy to occur under such working condition. The repeated starting and stopping of the air compression apparatus 10 affects its service life and increases heat generation. Therefore, an adjustment range can be preset for the rotation speed of the air compression apparatus 10, so as to enable the processor 20 to only be capable of adjusting the rotation speed of the air compression apparatus 10 within this adjustment range, that is, the processor 20 adjusts the rotation speed of the air compression apparatus 10 within a preset rotation speed range. A minimum rotation speed (a minimum value of a rotation speed range) can be preset to ensure that the air compression apparatus 10 maintains the pressure at the back end above the target pressure when the adjustment valve of fresh air is closed or has a small opening. This method can increase a service life of the air compression apparatus 10 and reduce heat generation, and at the same time solve the problem that the pump may not be started due to insufficient output torque, when starting under a certain backpressure. A minimum value of rotation speed range is preset, and the principle is that the air compression apparatus 10 will not shut down due to a too slow rotation speed when working at the minimum value of rotation speed range.

The inventors also found that since the output capacity of the air pump is not as good as that of a pipeline air, when an air supply is switched from the pipeline air to air pump supply, the previous setting value for flow amount may not be reached, which affects an oxygen concentration and a flow amount of an outputted fresh air. The anesthesia machine, that uses an electromagnetic valve to adjust the flow amount, adjusts through the electromagnetic valve to achieve a setting target for flow amount. When the setting value for flow amount of fresh air is within a maximum output capacity of the air pump, switching the air supply from pipeline air to the air pump does not affect a size of outputted flow amount. However, when the setting value for flow amount exceeds the maximum output capacity of the air pump, switching the air supply from pipeline air to the air pump affects the oxygen concentration and the flow amount of the outputted fresh air. For an anesthesia machine that adjusts a flow amount through a manual needle valve, if the air supply is switched from the pipeline air to the air pump, a fixed opening of the needle valve will inevitably cause a flow amount of the outputted fresh air to change. The same is true when the air supply is switched back from air pump to pipeline air.

Therefore, when a type of an air source for currently providing air is switched from the air interface to the air compression apparatus, the processor 20 may display corresponding prompt information through the human-machine interaction apparatus, thereby prompting the doctor that the air supply for supplying air is changed to the air compression apparatus. Similarly, the processor 20 may also display corresponding prompt information through the human-machine interaction apparatus when the type of the air source for currently providing air is switched from the air compression apparatus to the air interface. In this way, doctor will be more aware of possible change(s) in an air flow amount with this prompt information, and make corresponding adjustment(s) to avoid large fluctuation(s) in the air flow amount. Wherein, the air source are divided into two types: an air interface and an air compression apparatus.

The processor 20 can determine whether the type of the air source has been switched in a variety of ways. For example, when the processor 20 detects that the air compression apparatus 10 is turned on and the anesthesia machine is in working condition, it determines that the type of the air source has been switched from the air interface to the air compression apparatus. For another example, a pressure sensor or a flow amount sensor is provided at the air interface, and the processor 20 determines whether the type of the air source has been switched through a change of a value detected by the pressure sensor or the flow amount sensor. For example, when said value changes from a value which exceeds a preset value to 0 (indicating that the air interface is disconnected from the hospital pipeline), and the anesthesia machine is in working condition (for example, the air compression apparatus 10 is turned on), it is determined that the type of the air source has been switched from the air interface to the air compression apparatus. The preset value is a value greater than 0 to eliminate a pressure or flow change caused by wind. It only needs to be slightly greater than 0. The processor 20 may display corresponding prompt information through the human-machine interaction apparatus in a variety of ways. The following are a few examples to illustrate.

In one method, the processor 20 displays, through a human-machine interaction apparatus, the type of the air source which currently provides air. For example, if the air compression apparatus 10 is turned on, it means that the type of air source which currently provides air is an air compression apparatus. If the value detected by the pressure sensor or flow amount sensor at the air interface is greater than a preset value, it means that the type of air source which currently provides air is an air interface. This prompt method is simple and direct.

In the scheme of the above-mentioned embodiment 1, the first valve is usually an electromagnetic valve. After switching to the air compression apparatus 10 for supplying air, since the doctor presets a setting value for flow amount, even if an air flow amount changes, the processor 20 can automatically adjust the first valve according to the setting value for flow amount to maintain the air flow amount at the setting value for flow amount. In the solution of the above-mentioned embodiment 2, the second valve is manually controlled. Therefore, after the type of the air source is switched, the processor 20 can further prompt the user to adjust the second valve through the human-machine interaction apparatus. The doctor can adjust the second valve in time according to a possible change of the flow amount to avoid an adverse effect caused by switching of the type of the air source.

In the scheme of embodiment 2, after the processor 20 displays the corresponding prompt information through the human-machine interaction apparatus, it can also determine whether the characteristic quantity of opening degree for the second valve has changed. If the characteristic quantity of opening degree has changed, it means that the doctor has seen the prompt information and adjusted the second valve. Therefore, the purpose of the prompt has been achieved. When the characteristic quantity of opening degree for the second valve changes, the processor 20 stops the display of the information used to prompt the user to adjust the second valve. In this way, doctor do not need to manually close the prompt information, and the entire process is automated and intelligent, which is very convenient.

In the scheme of embodiment 2, if the detection apparatus uses a flow amount sensor to detect a current air flow amount of the second fresh gas branch 40, that is, the processor 20 detects an air flow amount through the flow amount sensor, and displays a current air flow amount (real-time air flow amount) detected by the flow amount sensor through the human-machine interaction apparatus. Then the processor 20 displays a corresponding prompt information through the human-machine interaction apparatus. Specifically, the processor 20 differentially displays air flow amounts detected by the flow amount sensor before and after the second valve is adjusted, on an interface of the human-machine interaction apparatus which interfaces displays air flow amount(s) detected by the flow amount sensor; such as changing a display color of a current air flow amount, highlighting a current air flow amount, etc. In short, the current air flow amount can be displayed more prominently. The doctor monitors the air flow amount provided by the second fresh gas branch through a real-time air flow amount on the display interface. When the doctor sees that a display mode of air flow amount has changed, the doctor knows that the type of the air source has been switched and the second valve may need to be adjusted. This prompt method does not take up additional space on the display interface.

In another way, in the scheme of embodiment 1, the processor 20 records the setting value for flow amount. After the type of the air source, which air source currently provides air, is switched from the air interface to the air compression apparatus, a difference between an outputted maximum flow amount of the air compression apparatus 10 and a target setting value for flow amount is determined according to said target setting value for flow amount when the air source type is the air interface (usually a current setting value for flow amount). When the outputted maximum flow amount is less than the target setting value for flow amount, the user is prompted to switch back from the air compression apparatus to the air interface through the human-machine interaction apparatus. The outputted maximum flow amount of the air compression apparatus 10 is a flow amount that the air compression apparatus 10 is capable of providing when working at a maximum output capacity (such as a maximum rotation speed), and is preset. That is, when the air pump cannot provide the target setting value for flow amount even when working at the maximum speed, the processor 20 reminds the user to switch back to a pipeline air supply to ensure high-flow air supply.

The above-mentioned embodiments all use valves to control the air flow amount. The following provides an embodiment for directly controlling an air flow amount of the second fresh gas branch through the air compression apparatus 10.

As shown in FIGS. 1 and 2, the anesthesia machine provided in this embodiment includes the first fresh gas branch 30 as described above, a second fresh gas branch 40, the third fresh gas branch 50 as described above, the detection apparatus 60 as described above, the anesthetic delivery apparatus 70 as described above, the respiratory loop 80 as described above, the ventilation control apparatus 90 as described above, the human-machine interaction apparatus as described above, and a processor 20.

In this embodiment, no valve for adjusting an air flow amount is provided at the second fresh gas branch 40, and the rest of the second fresh gas branch 40 is the same as the second fresh gas branch 40 of the above embodiment, which will not be described in detail here.

In addition to the functions mentioned in the above embodiments, the human-machine interaction apparatus is also used to receive a target setting value for flow amount which value is inputted by a user.

In addition to the functions mentioned in the above embodiments, the processor 20 is further configured to adjust an output capacity of the air compression apparatus 10 (such as adjusting an air flow amount which is provided by the air compression apparatus 10) according to the target setting value for flow amount. For example, flow amounts corresponding to different output capacities of the air compression apparatus 10 are calculated in advance to obtain a corresponding relationship between a flow amount and an output capacity. Then, the processor 20 adjusts an output capacity of the air compression apparatus 10 to an output capacity which corresponds to a target setting value for flow amount according to said target setting value for flow amount, thereby completing the setting of air flow amount. The processor 20 can specifically adjust the output capacity of the air compression apparatus by controlling an input voltage of the air compression apparatus 10. For example, a corresponding relationship between different input voltages and output capacities of the air compression apparatus (such as a rotation speed) is obtained in advance, thereby obtaining a corresponding relationship between a flow amount and an input voltage. The processor 20 applies an input voltage which corresponds to a target setting value for flow amount to the air compression apparatus 10, according to said target setting value for flow amount, thereby controlling the output capacity of the controller, such as controlling its rotation speed.

The detection apparatus 60 outputs a changing value for the air flow amount, which is provided by the air compression apparatus, following an adjustment for an output capacity of the air compression apparatus 10. For example, the detection apparatus 60 detects the air flow amount of the second fresh gas branch 40 in real time and displays the air flow amount through the human-machine interaction apparatus, so that the doctor can monitor the air flow amount in real time.

The anesthesia machine usually detects oxygen and/or nitrous oxide inside the first fresh gas branch and displays the oxygen and/or nitrous oxide on the display interface of the human-machine interaction apparatus. The air flow amount can be displayed together with flow amount(s) of oxygen and/or nitrous oxide. Considering the particularity of the type switching for the air source, the air flow amount can also be displayed independently of the flow amount(s) of oxygen and/or nitrous oxide, so that the doctor can monitor the air flow amount more attentively.

The ventilation control apparatus 90 controls the respiratory loop 80 to deliver the first mixed gas to the patient, which is specifically achieved through a drive gas. In the prior art, the drive gas also comes from a pipeline gas or a gas cylinder. In this disclosure, the drive gas may come from the aforementioned air compression apparatus 10 or from another air compression apparatus. The following are examples to illustrate this.

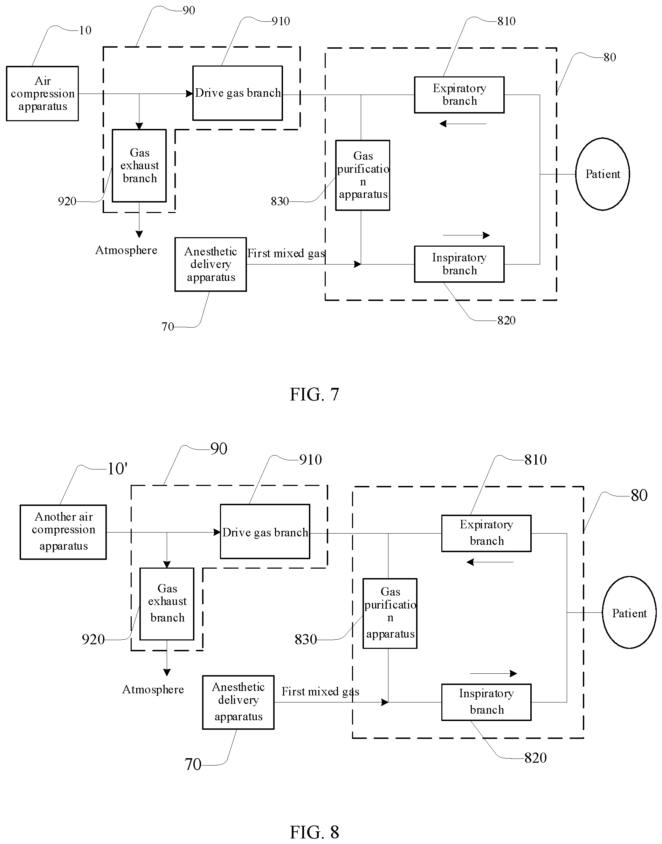

As shown in FIG. 7, in one embodiment, the air provided by the air compression apparatus 10 can also be used as drive gas for an anesthesia machine. For example, ventilation control apparatus 90 includes a drive gas branch 910. One end of the drive gas branch 910 is connected with the air compression apparatus 10, so as to obtain the air from the air compression apparatus 10, and the other end of the drive gas branch 910 is connected with the respiratory loop 80. The drive gas branch 910 is configured to use the air provided by the air compression apparatus 10 as the drive gas to periodically drive the respiratory loop 80 to deliver the first mixed gas to the patient.

Specifically, the respiratory loop 80 may include an expiratory branch 810, an inspiratory branch 820 and a gas purification apparatus 830. One end of the inspiratory branch 820 is connected with the output end of the anesthetic delivery apparatus 70 to receive the first mixed gas outputted by the anesthetic delivery apparatus 70. The other end of the inspiratory branch 820 is connected with a patient and is also connected with one end of the expiratory branch 810. The other end of the expiratory branch 810 is connected with the drive gas branch 910 and one end of the gas purification apparatus 830, and the other end of the gas purification apparatus 830 is connected with one end of the inspiratory branch 820. During an expiratory phase, the gas exhaled by the patient passes through the expiratory branch 810 and is stored in the drive gas branch 910; during an inspiratory phase, the drive gas branch 910 controls the drive gas to drive the exhaled gas stored in the drive gas branch 910 into the gas purification apparatus 830. For example, a valve arranged inside the drive gas branch 910 is opened, so as to enable the air outputted by the air compression apparatus 10 to serve as the drive gas for driving the exhaled gas stored in the drive gas branch 910 to enter the gas purification apparatus 830. The gas purification apparatus 830 removes at least part of carbon dioxide in the exhaled gas, so that the exhaled gas merges with the first mixed gas, and then enters into the inspiratory branch 820 under a drive of the drive gas, such that the purified exhaled gas and the first mixed gas are delivered to the patient.

The drive gas branch 910 may include a bellows including a shell and a folded bag located in the shell. There is a cavity between the shell and the folded bag. The folded bag is configured to store the gas exhaled by the patient. During the expiratory phase, the gas exhaled by the patient enters into the folded bag for storage. As an amount of stored gas increases, the folded bag expands, causing the volume of the cavity to decrease. In the inspiratory phase, the drive gas enters into the cavity, and the pressure in the cavity increases, thereby squeezing the exhaled gas stored in the folded bag into the respiratory loop 80.

Of course, instead of using a bellows, a volume reflector may be used, that is, the drive gas branch 910 may include a volume reflector. The volume reflector may be a thin and long pipe. In order to save space, the thin and long pipe may be bent into various shapes, such as a disc shape. The purpose of using a long and thin tube is to prevent the drive gas from entering the respiratory loop 80 as much as possible. The volume reflector is used to store the gas exhaled by the patient. During the expiratory phase, the gas exhaled by the patient enters into the volume reflector for storage. In the inspiratory phase, the drives gas enters into the volume reflector, thereby squeezing the exhaled gas stored in the volume reflector into the respiratory loop 80.

The ventilation control apparatus 90 may further include a gas exhaust branch 920, which is configured to exhaust an excess gas during the expiratory phase.

In one embodiment, the air outputted by the air compression apparatus 10 can be used as a fresh gas and a drive gas after pressure regulation. For example, the air compression apparatus 10 is connected with one end of the second fresh gas branch and the drive gas branch 910 through a pressure adjustment valve. Of course, in some embodiments, pressure adjustment valves may be provided in both the second fresh gas branch and the drive gas branch 910.

In the embodiment shown in FIG. 8, another air compression apparatus 10' is configured to provide the drive gas. For example, the ventilation control apparatus 90 includes a drive gas branch 910. The anesthesia machine further includes another air compression apparatus 10'. Said another air compression apparatus 10' is configured to provide air. Its structure and function are similar to those of the air compression apparatus in the aforementioned embodiment and will not be described in detail here. One end of the drive gas branch 910 is connected with said another air compression apparatus 10', and the other end of the drive gas branch 910 is connected with the respiratory loop 80. The drive gas branch 910 is configured to use air provided by another air compression apparatus 10' as the drive gas to periodically drive the respiratory loop 80 to deliver the first mixed gas to the patient.

The specific structure and connection relationship of the ventilation control apparatus 90 and the respiratory loop 80 are basically the same as those of the embodiment shown in FIG. 7, except that the air compression apparatus connected with the drive gas branch 910 is different, so they will not be described in detail here.

The air compression apparatuses 10 and 10' may be one of a turbine, an air pump and an air compression apparatus. For example, the air compression apparatus 10 may include an air pump, and said another air compression apparatus 10' may include a turbine.

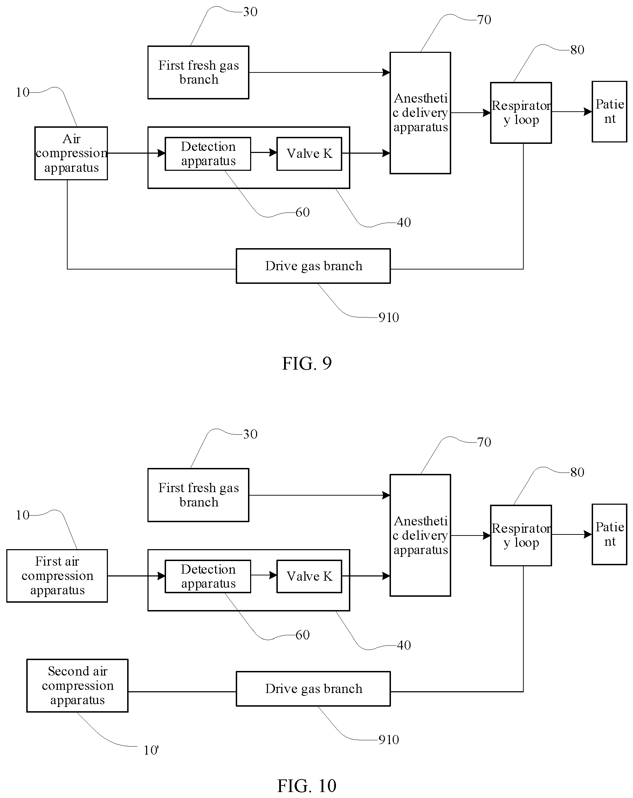

In some embodiments, the anesthesia machine provided by this disclosure may be as shown in FIG. 9, including: the first fresh gas branch 30 as described above, the second fresh gas branch 40 as described above, and the respiratory loop 80 as described above. The first fresh gas branch 30 is configured to provide oxygen and/or nitrous oxide. The second fresh gas branch 40 is connected with an air compression apparatus, and configured to provide air.

The anesthesia machine may further include a drive gas branch 910. One end of the drive gas branch 910 is connected with the air compression apparatus 10, and the other end of the drive gas branch 910 is connected with the respiratory loop 80. The drive gas branch 910 is configured to use air, which is provided by the air compression apparatus 10, as the drive gas to periodically drive the respiratory loop 80 to deliver the first mixed gas to the patient. The specific process is the same as the embodiment shown in FIG. 7, and will not be described in detail here.

The anesthesia machine may further include the processor 20 and the human-machine interaction apparatus as described above, as shown in the foregoing embodiments. Of course, the anesthesia machine may also include the third fresh gas branch 50 as described above, and its specific functions and connection relationships are shown in the aforementioned embodiments, which will not be described in detail here.

In still another embodiment, the anesthesia machine provided by this disclosure may be as shown in FIG. 10, including: the first fresh gas branch 30 as described above, the second fresh gas branch 40 as described above, and the respiratory loop 80 as described above. The first fresh gas branch 30 is configured to provide oxygen and/or nitrous oxide. The second fresh gas branch 40 is connected with a first air compression apparatus and is configured to provide air. The function of the first air compression apparatus is the same as that of the air compression apparatus 10 in the aforementioned embodiment, and will not be described in detail here.

The anesthesia machine may further include a drive gas branch 910 and a second air compression apparatus. The second air compression apparatus is also used to provide air. The function of the second air compression apparatus 10' is the same as that of said another air compression apparatus 10' in the aforementioned embodiment, and will not be described in detail here. One end of the drive gas branch 910 is connected with the second air compression apparatus, and the other end of the drive gas branch 910 is connected with the respiratory loop 80. The drive gas branch 910 is configured to use air provided by the second air compression apparatus as the drive gas to periodically drive the respiratory loop 80, so as to deliver the first mixed gas to the patient. The specific process is the same as the embodiment shown in FIG. 8, and will not be described in detail here.

It can be seen that in the anesthesia machine shown in FIGS. 7-10, even the drive gas can be generated by the anesthesia machine itself, thereby replacing the air cylinder, reducing a management risk, and also increasing an application scope of the anesthesia machine, making it more convenient to use.

This disclosure has been described with reference to various exemplary embodiments. However, those skilled in the art will recognize that changes and modifications may be made to the exemplary embodiments without departing from the scope of this disclosure. For example, the various operational steps and components for performing the operational steps may be implemented in different ways (e.g., one or more steps may be deleted, modified, or combined into other steps) depending on the specific application or considering any number of cost functions associated with the operation of the system.

Additionally, as will be appreciated by those skilled in the art, the principles of this disclosure may be reflected in a computer program product on a computer-readable storage medium pre-installed with computer-readable program code. Any tangible, non-transitory computer-readable storage medium may be used, including a magnetic storage apparatus (hard disk, floppy disk, etc.), an optical storage apparatus (CD-ROM, DVD, Blu Ray disk, etc.), a flash memory, and/or the likes. These computer program instructions may be loaded onto a general-purpose computer, a special-purpose computer or other programmable data processing apparatus to form a machine, so that these instructions executed on the computer or other programmable data processing apparatus may generate means for implementing the specified functions. These computer program instructions may also be stored in a computer-readable memory, which may instruct a computer or other programmable data processing apparatus to operate in a specific manner, such that the instructions stored in the computer-readable memory may form an article of manufacture, including an implementation apparatus that implements the specified functions. Computer program instructions may also be loaded onto a computer or other programmable data processing apparatus so that a series of operational steps are performed on the computer or other programmable apparatus to produce a computer-implemented process, so that the instructions executed on the computer or other programmable apparatus may provide steps for implementing specified functions.

Although the principles herein have been illustrated in various embodiments, many modifications of structure, arrangement, proportions, elements, materials, and components specifically adapted to particular environments and operating requirement may be used without departing from the principles and scope of this disclosure. The above modifications and other changes or corrections are intended to be included within the scope of this disclosure.

The foregoing detailed description has been described with reference to various embodiments. However, one skilled in the art will appreciate that various modifications and changes can be made without departing from the scope of this disclosure. Accordingly, this disclosure is to be considered in an illustrative rather than a restrictive sense, and all such modifications are intended to be included within the scope thereof. Likewise, advantages, other advantages, and solutions to problems have been described above with regard to various embodiments. However, benefits, advantages, solutions to problems, and any elements that produce the same, or render them more evident, should not be construed as critical, required, or essential. As used herein, the term "includes," and any other variations thereof, are intended to be non-exclusive inclusions, such that a process, method, article, or apparatus that includes a list of elements includes not only those elements but also other elements that are not expressly listed or that do not belong to the process, method, system, article, or apparatus. Furthermore, the term "coupled" and any other variations thereof as used herein refers to a physical, electrical, magnetic, optical, communicative, functional, and/or any other connection.

**Claims**

1. An anesthesia machine, **characterized in that**, comprising:

   a first fresh gas branch, which is configured to provide oxygen and/or nitrous oxide;
   a second fresh gas branch, which is connected with an air compression apparatus and configured to provide air; wherein an air flow amount which is provided by the air compression apparatus is adjustable;
   wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;

a respiratory loop, which is configured to receive the first mixed gas which is outputted by the anesthetic delivery apparatus, and deliver the first mixed gas to a patient;

a detection apparatus, which is configured to detect a characteristic quantity which characterizes an air flowing characteristic of the second fresh gas branch; and

a processor, which is configured to adjust the air flow amount which is provided by the air compression apparatus, according to the characteristic quantity which is outputted by the detection apparatus.

2.  The anesthesia machine according to claim 1, **characterized in that**, when the characteristic quantity is a current air flow amount of the second fresh gas branch, in order to adjust the air flow amount which is provided by the air compression apparatus, according to the characteristic quantity which is outputted by the detection apparatus, the processor is specifically configured to: obtain a current setting value for flow amount of the second fresh gas branch; and adjust the air flow amount which is provided by the air compression apparatus, according to a difference between the current air flow amount and the current setting value for flow amount; or

when the characteristic quantity does not comprise a current air flow amount of the second fresh gas branch, in order to adjust the air flow amount which is provided by the air compression apparatus, according to the characteristic quantity which is outputted by the detection apparatus, the processor is specifically configured to: obtain a current air flow amount of the second fresh gas branch according to the characteristic quantity which is outputted by the detection apparatus; obtain a current setting value for flow amount of the second fresh gas branch; and adjust the air flow amount which is provided by the air compression apparatus, according to a difference between the current air flow amount and the current setting value for flow amount.

3.  The anesthesia machine according to claim 2, **characterized in that**, adjusting the air flow amount which is provided by the air compression apparatus, comprises: adjusting a rotation speed of the air compression apparatus;

the detection apparatus comprises a first flow amount sensor, wherein the first flow amount sensor is configured to detect the current air flow amount of the second fresh gas branch; the current air flow amount which is detected by the first flow amount sensor is used as the characteristic quantity;

the processor is further configured to obtain the current air flow amount which is detected by the first flow amount sensor, so as to adjust the rotation speed of the air compression apparatus.

4.  The anesthesia machine according to claim 2, **characterized in that**, further comprising a third fresh gas branch and an oxygen concentration sensor; wherein the first fresh gas branch is specifically configured to provide the oxygen, the third fresh gas branch is configured to mix the oxygen which is provided by the first fresh gas branch and the air which is provided by the second fresh gas branch, so as to obtain a second mixed gas; the oxygen concentration sensor is configured to detect an oxygen concentration of the second mixed gas;

the detection apparatus comprises a second flow amount sensor, wherein the second flow amount sensor is configured to detect a flow amount of the oxygen which is provided by the first fresh gas branch or a flow amount of the second mixed gas which is provided by the third fresh gas branch;

adjusting the air flow amount which is provided by the air compression apparatus, comprises: adjusting a rotation speed of the air compression apparatus;

the processor is further configured to obtain the current air flow amount of the second fresh gas branch, according to the oxygen concentration of the second mixed gas and the flow amount of the oxygen or according to the oxygen concentration of the second mixed gas and the flow amount of the second mixed gas, so as to adjust the rotation speed of the air compression apparatus.

5.  The anesthesia machine according to claim 2, **characterized in that**, in order to adjust the air flow amount which is provided by the air compression apparatus, according to a difference between the current air flow amount and the current setting value for flow amount, the processor is specifically configured to:

increase the air flow amount which is provided by the air compression apparatus, when the current air flow amount is lower than the current setting value for flow amount; or

decrease the air flow amount which is provided by the air compression apparatus, when the current air flow amount is higher than the current setting value for flow amount.

**6.** The anesthesia machine according to claim 2, **characterized in that**, further comprising a human-machine interaction apparatus; wherein in order to obtain a current setting value for flow amount, the processor is specifically configured to:
obtain, through the human-machine interaction apparatus, the current setting value for flow amount which is inputted by a user.

**7.** The anesthesia machine according to claim 2, **characterized in that**, further comprising a first valve which is arranged inside the second fresh gas branch, wherein the first valve is configured to adjust the current air flow amount of the second fresh gas branch;
wherein the processor is further configured to:

adjust an opening degree of the first valve and then adjust the air flow amount which is provided by the air compression apparatus, according to the difference between the current air flow amount and the current setting value for flow amount; or
simultaneously adjust an opening degree of the first valve and the air flow amount which is provided by the air compression apparatus, according to the difference between the current air flow amount and the current setting value for flow amount; or
adjust the air flow amount which is provided by the air compression apparatus and then adjust an opening degree of the first valve, according to the difference between the current air flow amount and the current setting value for flow amount.

**8.** The anesthesia machine according to claim 1, **characterized in that**, the first valve is an electromagnetic valve.

**9.** The anesthesia machine according to claim 1, **characterized in that**, further comprising a second valve, which is arranged inside the second fresh gas branch and configured to support a user to manually adjust an air flow amount of the second fresh gas branch;
in order to adjust the air flow amount which is provided by the air compression apparatus, according to the characteristic quantity which is outputted by the detection apparatus, the processor is specifically configured to:
adjust the air flow amount which is provided by the air compression apparatus, according to a change of the characteristic quantity, which change is caused by a user manually adjusting the second valve.

**10.** The anesthesia machine according to claim 9, **characterized in that**, further comprising a third flow amount sensor; wherein the third flow amount sensor is configured to detect an air flow amount of the second fresh gas branch; the characteristic quantity is the air flow amount of the second fresh gas branch; preferably, in order to adjust the air flow amount which is provided by the air compression apparatus, according to a change of the characteristic quantity, which change is caused by a user manually adjusting the second valve, the processor is specifically configured to:

increase the air flow amount which is provided by the air compression apparatus, when the air flow amount which is detected by the third flow amount sensor is increased; and/or
decrease the air flow amount which is provided by the air compression apparatus, when the air flow amount which is detected by the third flow amount sensor is decreased.

**11.** The anesthesia machine according to claim 9, **characterized in that**, the characteristic quantity comprises a characteristic quantity of opening degree for the second valve;
in order to adjust the air flow amount which is provided by the air compression apparatus, according to the characteristic quantity which is outputted by the detection apparatus, the processor is specifically configured to:

increase the air flow amount which is provided by the air compression apparatus, when the characteristic quantity of opening degree is increased; and/or
decrease the air flow amount which is provided by the air compression apparatus, when the characteristic quantity of opening degree is decreased.

**12.** The anesthesia machine according to claim 11, **characterized in that**, the detection apparatus comprises a first pressure sensor, wherein the first pressure sensor is configured to detect an air pressure of the second fresh gas branch at a front end of the second valve; the characteristic quantity of opening degree for the second valve is the air pressure;
preferably, in order to increase the air flow amount which is provided by the air compression apparatus, when the characteristic quantity of opening degree is increased; and/or decrease the air flow amount which is provided by the air compression apparatus, when the characteristic quantity of opening degree is decreased, the processor is specifically configured to:

increase the air flow amount which is provided by the air compression apparatus, when the air pressure is lower than a target pressure; and/or decrease the air flow amount which is provided by the air compression apparatus, when the air pressure is higher than a target pressure; or increase the air flow amount which is provided by the air compression apparatus, when the air pressure is decreased; and/or decrease the air flow amount which is provided by the air compression apparatus, when the air pressure is increased.

13. The anesthesia machine according to claim 11, **characterized in that**, the detection apparatus comprises a second pressure sensor, a third flow amount sensor and a third pressure sensor;

the third flow amount sensor is configured to detect the air flow amount of the second fresh gas branch; the second pressure sensor is configured to detect an air pressure of the second fresh gas branch at a front end of the second valve; the third pressure sensor is configured to detect an air pressure of the second fresh gas branch at a back end of the second valve; the air pressure which is detected by the second pressure sensor, the air flow amount which is detected by the third flow amount sensor, and the air pressure which is detected by the third pressure sensor, are capable of being used to calculate the characteristic quantity of opening degree for the second valve.

14. The anesthesia machine according to claim 11, **characterized in that**, the detection apparatus comprises a displacement sensor or a gap sensor, wherein the displacement sensor or the gap sensor is configured to detect an opening degree for the second valve, so as to obtain the characteristic quantity of opening degree for the second valve.

15. The anesthesia machine according to claim 1, **characterized in that**, further comprising an air interface and a human-machine interaction apparatus; wherein the air interface is configured to receive air provided from outside; one of the air interface and the air compression apparatus are connected with the second fresh gas branch; wherein the processor is further configured to: display corresponding prompt information through the human-machine interaction apparatus, when a type of an air source, which air source currently provides air, is switched from the air interface to the air compression apparatus and/or from the air compression apparatus to the air interface; wherein the type of the air source is divided into two types, in which one type is the air interface and the other is the air compression apparatus.

16. The anesthesia machine according to claim 15, **characterized in that**, in order to display corresponding prompt information through the human-machine interaction apparatus, the processor is specifically configured to:

display, through the human-machine interaction apparatus, the type of the air source, which air source currently provides air; and/or after the type of the air source, which air source currently provides air, is switched from the air interface to the air compression apparatus, prompt a user, through the human-machine interaction apparatus, to switch back from the air compression apparatus to the air interface, when a maximum flow amount, which is outputted by the air compression apparatus, is determined to be less than a target setting value for flow amount, according to the target setting value for flow amount.

17. The anesthesia machine according to claim 15, **characterized in that**, further comprising a second valve which is configured to support a user to manually adjust an air flow amount of the second fresh gas branch; in order to display corresponding prompt information through the human-machine interaction apparatus, the processor is specifically configured to: prompt a user to adjust the second valve through the human-machine interaction apparatus.

18. The anesthesia machine according to claim 17, **characterized in that**, the characteristic quantity comprises a characteristic quantity of opening degree for the second valve; the processor is further configured to: determine whether the characteristic quantity of opening degree for the second valve changes; and automatically stop displaying information to prompt a user to adjust the second valve, when the characteristic quantity of opening degree for the second valve changes.

19. The anesthesia machine according to claim 18, **characterized in that**, the detection apparatus comprises a first flow amount sensor; wherein the first flow amount sensor is configured to detect the air flow amount of the second fresh gas branch; in order to display corresponding prompt information through the human-machine interaction apparatus, the processor is specifically configured to: differentially display air flow amounts which are respectively detected by the first flow amount sensor before and after adjusting the second valve.

20. An anesthesia machine, **characterized in that**, comprising:

a first fresh gas branch, which is configured to provide oxygen and/or nitrous oxide;

a second fresh gas branch, which is connected with an air compression apparatus and configured to provide air; wherein an air flow amount which is provided by the air compression apparatus is adjustable;

wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;

a respiratory loop, which is configured to receive the first mixed gas which is provided by the anesthetic delivery apparatus;

a ventilation control apparatus, which is configured to control the respiratory loop to deliver the first mixed gas to a patient, so as to provide an anesthesia respiratory support for the patient;

a human-machine interaction apparatus, which is configured to receive a target setting value for flow amount;

a processor, which is configured to adjust the air flow amount which is provided by the air compression apparatus, according to the target setting value for flow amount.

21. The anesthesia machine according to claim 20, **characterized in that**, no valve for adjusting an air flow amount of the second fresh gas branch is arranged at the second fresh gas branch; wherein the anesthesia machine further comprises a detection apparatus, which is configured to detect the air flow amount of the second fresh gas branch; wherein the detection apparatus is further configured to output a changing value for the air flow amount which is provided by the air compression apparatus, following an adjustment for an output capacity of the air compression apparatus.

22. The anesthesia machine according to any one of claims 1~21, **characterized in that**, the processor is further configured to adjust the air flow amount which is provided by the air compression apparatus by controlling an input voltage of the air compression apparatus.

23. The anesthesia machine according to any one of claims 1~21, **characterized in that**, the air compression apparatus is arranged inside a housing of the anesthesia machine.

24. The anesthesia machine according to any one of claims 1~21, **characterized in that**, the air compression apparatus comprises an air pump with an adjustable rotation speed, wherein the air pump is configured to compress and then output air which enters from an air inlet;

wherein the anesthesia machine further comprises the air inlet; a first filter is arranged between the air inlet and the air pump, wherein the first filter is configured to filter said air which enters from the air inlet; preferably, the second fresh gas branch is provided with a second filter, and the second filter is configured to filter air which is outputted by the air pump.

25. The anesthesia machine according to claim 24, **characterized in that**, a normally closed pressure-relief branch is arranged at the second fresh gas branch, wherein the normally closed pressure-relief branch comprises a normally closed overflow valve, which is arranged at a back end of the air pump; wherein the normally closed overflow valve is configured to open the normally closed pressure-relief branch, when an air pressure of the second fresh gas branch is higher than a work pressure, so as to enable air inside the second fresh gas branch to flow out from the normally closed pressure-relief branch.

26. The anesthesia machine according to any one of claims 1~21, **characterized in that**, adjusting the air flow amount which is provided by the air compression apparatus, comprises: adjusting a rotation speed of the air compression apparatus;

the processor is further configured to adjust the rotation speed of the air compression apparatus within a preset rotation speed range; the air compression apparatus will not shut down due to a too-slow rotation speed, when working at a minimum value of the rotation speed range.

27. An anesthesia machine, **characterized in that**, comprising:

a first fresh gas branch, which is configured to provide oxygen and/or nitrous oxide;

a second fresh gas branch, which is connected with an air compression apparatus and configured to provide air; wherein an air flow amount which is provided by the air compression apparatus is adjustable;

a second valve, which is configured to support a user to manually adjust an air flow amount which is provided by the second fresh gas branch;

wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;

a respiratory loop, which is configured to receive the first mixed gas which is outputted by the anesthetic delivery apparatus, and deliver the first mixed gas to a patient;

a detection apparatus, which is configured to detect a characteristic quantity of opening degree for the second valve; and

a processor, which is configured to adjust an output capacity of the air compression apparatus, according to a change of the characteristic quantity of opening degree.

28. The anesthesia machine according to any one of claims **1,** 20 or 27, **characterized in that**, further comprising:

a drive gas branch, wherein one end of the drive gas branch is connected with the air compression apparatus, and the other end of the drive gas branch is connected with the respiratory loop; wherein the drive gas branch is configured to use air, which is provided by the air compression apparatus, as a drive gas, so as to periodically drive the respiratory loop to deliver the first mixed gas to a patient; or

a drive gas branch and another air compression apparatus, wherein one end of the drive gas branch is connected with said another air compression apparatus, and the other end of the drive gas branch is connected with the respiratory loop; wherein the drive gas branch is configured to use air, which is provided by said another air compression apparatus, as a drive gas, so as to periodically drive the respiratory loop to deliver the first mixed gas to a patient.

29. An anesthesia machine, **characterized in that**, comprising:

a first fresh gas branch, which is configured to provide oxygen and/or nitrous oxide;

a second fresh gas branch, which is connected with an air compression apparatus and configured to provide air;

wherein the first fresh gas branch and the second fresh gas branch are both connected with an anesthetic delivery apparatus, wherein the anesthetic delivery apparatus is configured to mix anesthetic, the oxygen and/or nitrous oxide which are/is provided by the first fresh gas branch, and the air which is provided by the second fresh gas branch, so as to obtain a first mixed gas;

a respiratory loop, which is configured to receive the first mixed gas which is outputted by the anesthetic delivery apparatus, and deliver the first mixed gas to a patient; and
a drive gas branch;

wherein one end of the drive gas branch is connected with the air compression apparatus, and the other end of the drive gas branch is connected with the respiratory loop; wherein the drive gas branch is configured to use air, which is provided by the air compression apparatus, as a drive gas, so as to periodically drive the respiratory loop to deliver the first mixed gas to a patient;

or, the anesthesia machine further comprises another air compression apparatus, wherein one end of the drive gas branch is connected with said another air compression apparatus, and the other end of the drive gas branch is connected with the respiratory loop; wherein the drive gas branch is configured to use air, which is provided by said another air compression apparatus, as a drive gas, so as to periodically drive the respiratory loop to deliver the first mixed gas to a patient.

30. The anesthesia machine according to claim 29, **characterized in that**, the air compression apparatus comprises a turbine, an air pump or an air compressor.

31. The anesthesia machine according to claim 30, **characterized in that**, the air compression apparatus comprises an air pump, and said another air compression apparatus comprises a turbine.

32. The anesthesia machine according to claim 30, **characterized in that**, the air compression apparatus is in respective connection with one end of the second fresh gas branch and one end of the drive gas branch, through one pressure adjustment valve; or

the second fresh gas branch and the drive gas branch are respectively provided with a pressure adjustment valve.

FIG. 1

FIG. 2

110

P0

120    130    140

150

160

FIG. 3

Control a respiratory loop to deliver a first mixed gas to a patient                    1

Detect a characteristic quantity which characterizes an air flowing characteristic of the second fresh gas branch    2

Adjust an air flow amount which is provided by an air compression apparatus, according to the characteristic quantity    3

FIG. 4

31

| Determine a current air flow amount of a second fresh gas branch |
|---|

32

| Obtain a current setting value for flow amount |
|---|

33

| Adjust an output capacity of an air compression apparatus according to a difference between a current air flow amount and a current setting value for flow amount |
|---|

FIG. 5

10    (P2)    610    (P3)    70    80

| Air compression apparatus | → | Third fresh gas branch | → | Anesthetic delivery apparatus | → | Respiratory loop | → | Patient |
|---|---|---|---|---|---|---|---|---|

| Opening degree calculation unit | 620 |
|---|---|

FIG. 6

FIG. 7

FIG. 8

FIG. 9

FIG. 10

# INTERNATIONAL SEARCH REPORT

| International application No. |
|---|
| **PCT/CN2024/098486** |

| A. | CLASSIFICATION OF SUBJECT MATTER |
|---|---|

A61M16/01(2006.01)i; A61M16/00(2006.01)i

According to International Patent Classification (IPC) or to both national classification and IPC

| B. | FIELDS SEARCHED |
|---|---|

Minimum documentation searched (classification system followed by classification symbols)

IPC: A61M

Documentation searched other than minimum documentation to the extent that such documents are included in the fields searched

Electronic data base consulted during the international search (name of data base and, where practicable, search terms used)

CNTXT; WPABSC; ENTXTC; CNKI; VEN; ENTXT; WPABS: 迈瑞, 蔡琨, 鲍旺, 陈轶炜, 于文浩, 麻醉, 麻药, 空压机, 压缩机, 空气压缩, 泵, 风机, 风扇, 载气, 驱动, 阀, 开度, 新鲜气体, 笑气, 流量, 流速, 压力, 转速, 溢流, 过压, 超压, 泄压, respirat+, ventilat+, anesthesia, compress+, fan, pump, driv+, valve, flow, rate, pressure

| C. | DOCUMENTS CONSIDERED TO BE RELEVANT |
|---|---|

| Category* | Citation of document, with indication, where appropriate, of the relevant passages | Relevant to claim No. |
|---|---|---|
| X | US 2023181863 A1 (DRAEGERWERK AG & CO., KGAA) 15 June 2023 (2023-06-15) description, paragraphs [0082]-[0125], and figures 1-7 | 1-27 |
| Y | US 2023181863 A1 (DRAEGERWERK AG & CO., KGAA) 15 June 2023 (2023-06-15) description, paragraphs [0082]-[0125], and figures 1-7 | 28-32 |
| X | US 2018221606 A1 (DRAEGERWERK AG & CO., KGAA) 09 August 2018 (2018-08-09) description, paragraphs [0040]-[0067], and figures 1-6 | 1-27 |
| Y | US 2018221606 A1 (DRAEGERWERK AG & CO., KGAA) 09 August 2018 (2018-08-09) description, paragraphs [0040]-[0067], and figures 1-6 | 28-32 |
| Y | WO 2020087397 A1 (SHENZHEN MINDRAY BIOMEDICAL ELECTRONICS CO., LTD. et al.) 07 May 2020 (2020-05-07) description, page 4, line 16 to page 13, line 6, and figures 1-4 | 28-32 |
| A | US 5848591 A (DRAEGERWERK AG) 15 December 1998 (1998-12-15) entire document | 1-32 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **14 September 2024** | **19 September 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| :--- |
| **PCT/CN2024/098486** |

**C.    DOCUMENTS CONSIDERED TO BE RELEVANT**

| Category* | Citation of document, with indication, where appropriate, of the relevant passages | Relevant to claim No. |
| :---: | :--- | :---: |
| A | US 2008121233 A1 (DRAEGER MEDICAL AG & CO., KGAA) 29 May 2008 (2008-05-29)<br>entire document | 1-32 |
| A | CN 106470725 A (KAGAN EUGEN) 01 March 2017 (2017-03-01)<br>entire document | 1-32 |
| A | CN 111511430 A (GENERAL ELECTRIC COMPANY) 07 August 2020 (2020-08-07)<br>entire document | 1-32 |
| A | CN 103096960 A (IMT AG) 08 May 2013 (2013-05-08)<br>entire document | 1-32 |
| A | CN 114642810 A (SHENZHEN AMOUL TECHNOLOGY CO., LTD.) 21 June 2022<br>(2022-06-21)<br>entire document | 1-32 |

Form PCT/ISA/210 (second sheet) (July 2022)

EP 4 596 011 A1

## INTERNATIONAL SEARCH REPORT
### Information on patent family members

International application No.

**PCT/CN2024/098486**

| Patent document cited in search report | | | Publication date (day/month/year) | Patent family member(s) | | | Publication date (day/month/year) |
|---|---|---|---|---|---|---|---|
| US | 2023181863 | A1 | 15 June 2023 | None | | | |
| US | 2018221606 | A1 | 09 August 2018 | US | 11141551 | B2 | 12 October 2021 |
| WO | 2020087397 | A1 | 07 May 2020 | None | | | |
| US | 5848591 | A | 15 December 1998 | None | | | |
| US | 2008121233 | A1 | 29 May 2008 | US | 8047205 | B2 | 01 November 2011 |
| CN | 106470725 | A | 01 March 2017 | None | | | |
| CN | 111511430 | A | 07 August 2020 | None | | | |
| CN | 103096960 | A | 08 May 2013 | None | | | |
| CN | 114642810 | A | 21 June 2022 | None | | | |

Form PCT/ISA/210 (patent family annex) (July 2022)